(19) **Europäisches Patentamt**
**European Patent Office**
**Office européen des brevets**

(11) **EP 3 599 973 B1**

(12) **EUROPEAN PATENT SPECIFICATION**

(45) Date of publication and mention
of the grant of the patent:
**22.01.2025 Bulletin 2025/04**

(21) Application number: **18725246.5**

(22) Date of filing: **23.05.2018**

(51) International Patent Classification (IPC):
**A61B 1/00** *(2006.01)*    **A61B 1/04** *(2006.01)*
**A61B 5/07** *(2006.01)*

(52) Cooperative Patent Classification (CPC):
**A61B 1/041; A61B 1/00158; A61B 5/073;**
A61B 10/0283; A61B 2010/0061

(86) International application number:
**PCT/EP2018/063530**

(87) International publication number:
**WO 2018/219741 (06.12.2018 Gazette 2018/49)**

(54) **MAGNETICALLY ACTUATED CAPSULE ENDOSCOPE, MAGNETIC FIELD GENERATING AND SENSING APPARATUS AND METHOD OF ACTUATING A MAGNETICALLY ACTUATED CAPSULE ENDOSCOPE**

MAGNETISCH BETÄTIGTES KAPSELENDOSKOP, MAGNETFELDERZEUGUNGS- UND -MESSVORRICHTUNG UND VERFAHREN ZUR BETÄTIGUNG EINES MAGNETISCH BETÄTIGTEN KAPSELENDOSKOPS

ENDOSCOPE À CAPSULE À ACTIONNEMENT MAGNÉTIQUE, APPAREIL DE GÉNÉRATION ET DE DÉTECTION DE CHAMP MAGNÉTIQUE ET PROCÉDÉ D'ACTIONNEMENT D'UN ENDOSCOPE À CAPSULE À ACTIONNEMENT MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2017 EP 17173196**

(43) Date of publication of application:
**05.02.2020 Bulletin 2020/06**

(73) Proprietor: **Max-Planck-Gesellschaft zur Förderung der Wissenschaften e.V.**
**80539 München (DE)**

(72) Inventors:
• **SITTI, Metin**
**70569 Stuttgart (DE)**
• **SON, Donghoon**
**Daegu 42261 (KR)**

(74) Representative: **Manitz Finsterwald**
**Patent- und Rechtsanwaltspartnerschaft mbB**
**Martin-Greif-Strasse 1**
**80336 München (DE)**

(56) References cited:
**WO-A2-2009/060460**    **JP-A- 2008 018 254**
**US-A1- 2011 065 987**    **US-A1- 2011 166 416**
**US-A1- 2013 303 847**

**Description**

The present invention relates to a magnetically actuated capsule endoscope, the endoscope comprising: a body having a top housing part and a bottom housing part; a permanent magnet arranged within the top housing part; a plurality of deformable members extending from the top housing part to an end of the bottom housing part; and a needle projecting from the top housing part towards the bottom housing part and surrounded by the plurality of deformable members, wherein the plurality of deformable members are configured to repeatedly expand and contract to respectively cover and uncover a tip of said needle. A magnetic field generating and sensing apparatus for a magnetically actuated endoscope and a method of actuating a magnetically actuated capsule endoscope, not being part of the invention, have been also disclosed.

Current medical technologies are converging to minimally invasive diagnosis and therapy. In the diagnosis of gastrointestinal (GI) diseases, the efforts to reduce patient discomfort have resulted in the development of capsule endoscopes. Wireless capsule endoscopes, in the form of a pill, are well tolerated by patients, and they are currently used to collect diagnostic images and videos for the identification of suspicious lesions. They have even become the standard of care for some diagnostic procedures, such as the investigation of obscure GI bleeding. While capsule endoscopy has already begun to revolutionize the diagnosis of GI disorders in the clinic, the possibilities afforded by capsule endoscopy are still being explored. One of the major limitations of commercially available devices is their inability to take biopsy samples after a suspicious lesion is identified. Acquiring a biopsy currently requires an additional endoscopic procedure, adding to the overall time and expense of making an accurate diagnosis. The ability to take biopsy samples is therefore a highly desirable addition to the feature set of capsule endoscopes.

Several capsule designs for taking biopsy samples have already been presented. The idea of taking biopsy samples in the GI tract with a small, swallowable device in the form of a capsule appeared as early as 1967 when an electromagnetic biopsy device was first presented. The capsule utilizes the peristalsis of the lower GI tract, air pressure through a tether, and an electromagnetically actuated razor inside the robot for cutting tissue.

Furthermore, a rotational micro biopsy device was developed that was capable of cutting sample tissues using a rotational razor inside the capsule, utilizing torsional springs (thermally or SMA triggered). Other suggestions have been put forward suggesting the use of forceps or cutting razor-style biopsy tools through a narrow hole on the side of the capsule. Other forceps-style biopsy tools that have been suggested are actuated by a torsion spring, using a shape-memory alloy trigger or are driven by a coil-based wireless power transfer system with double-balloon-enteroscopy-style locomotion. For example, a magnetic torsion spring was integrated in a biopsy capsule that enables a blade to cut a tissue when the external magnetic field is removed.

Although most of the biopsy capsule designs have been created with lower GI tract applications in mind, it is possible that some of them may additionally work in the upper GI tract. One previous design has been specifically investigated for upper GI tract procedures, in this connection a magnetically actuated soft capsule endoscope (MASCE) with micro-grippers was used. MASCE carries and deploys thermosensitive micro-grippers in the stomach and retrieves them with an adhesive patch after the micro-grippers grab the stomach tissues using its self-folding mechanism. However, experiments indicated that the yield of micro-gripper retrieval is low (3%), and in some cases a physician may prefer a targeted biopsy to the stochastic sampling performed by the micro-grippers.

Regardless of the location in the GI tract, one of the main limitations of all prior designs is that they can only take superficial biopsy samples. Those are useful to retract the tissues on the mucosal layer of the GI tract, however, it is difficult to reach deep masses inside the GI tract. Those mechanisms can miss submucosal tumors (SMTs), which can lower the diagnostic accuracy.

Prior art devices are known from the following documents US 2013/303847 A1; US 2011/166416 A1; US 2011/065987 A1; JP 2008 018254 A; and WO 2009/060460 A2.

For this reason it is an object of the present invention to obtain a capsule endoscope that can be maneuvered within at least the upper GI tract or stomach that can record images indicating a state of the upper GI tract or stomach and that is capable of reliably performing a biopsy even of deep lying masses if a lesion or tumor is assumed on the basis of the images recorded by the endoscope.

This object is satisfied by a magnetically actuated capsule endoscope having the features of claim 1.

Such a magnetically actuated capsule endoscope preferably comprises:

a body having a top housing part and a bottom housing part;
a permanent magnet arranged within the top housing part;
a plurality of deformable members extending from the top housing part to an end of the bottom housing part; and
a needle projecting from the top housing part towards the bottom housing part and surrounded by the plurality of deformable members, wherein the plurality of deformable members are configured to repeatedly expand and contract to respectively cover and uncover a tip of said needle.

In this way a novel magnetically actuated soft capsule endoscope with fine-needle aspiration biopsy (FNAB) functionality called B-MASCE is made available. FNAB is a type of biopsy technique to extract tissue samples using a fine needle. It was reported that fine-needle aspiration biopsy technique provides better accuracy than other biopsy techniques like forceps or brush, with the absence of complications in the GI tract. Because the needle can penetrate deep inside the mass of a lesion, this technique improves diagnostic accuracy even in the case of SMTs.

To this end a thin and hollow needle is attached to the capsule, which can penetrate deeply into tissues to obtain subsurface biopsy sample. The design utilizes a soft elastomer body as a compliant mechanism to guide the needle on the application of an external magnetic field causing the deformable member to contract and to thereby cause the top housing part move relative to the bottom housing part causing the needle to move relative to the bottom housing part while it is fixed at the top housing part. An internal permanent magnet provides a means for both actuation and tracking. The capsule is designed to roll towards its target and then deploy the biopsy needle in a precise location selected as the target area.

B-MASCE is controlled by multiple custom-designed electromagnets while its position and orientation are tracked by a magnetic sensor array. In in vitro trials, B-MASCE demonstrated rolling locomotion and biopsy of a swine tissue model positioned inside an anatomical human stomach model. It was confirmed after the experiment that a tissue sample was retained inside the needle.

The design of B-MASCE enables both the required motion for FNAB (axial jabbing motion of the needle) and rolling locomotion on the surface of a stomach. It is controlled by a magnetic force and torque on an internal magnet with external magnetic field control. Soft material-based legs (deformable members) connecting the two ends of the capsule work as a spring and a guide for the needle. The design is based on the clinical data suggesting the appropriate needle gauge and penetration depth for FNAB. In vitro experiments and a demonstration were performed with a swine tissue model, and it was verified that a biopsy sample was successfully captured.

Fine-needle aspiration biopsy technique, also known as fine-needle aspiration cytology, describes the method of aspirating cells from suspicious tissue lesions through a thin and hollow needle. During the procedure, a hollow needle is inserted into a mass to extract samples of tissue. Once removed, they are sent to the laboratory for further examination. It is commonly used to investigate lumps located in the thyroid or the breast, and it has also been successfully applied in the GI tract.

The integrated fine-needle at the upper part of the robot can penetrate into a lesion and capture samples inside the hollow needle using a technique similar to standard clinical procedure. A permanent magnet inside the robot generates a magnetic force and torque through its interaction with an external magnetic field, and it is simultaneously used as a tracking source at the same time. The soft material-based Sarrus linkage of the deformable members works as a needle guide, spring, and body for the capsule. This allows the robot to collapse, deploying the needle into the targeted tissue, and also provides the restoration force for the needle retraction when the external magnetic field is removed.

Advantageously the end of the bottom housing part further comprises a locating pad, optionally wherein the locating pad has a concave shape at the end of the bottom housing part.

The locating pad can be utilised to facilitate the attachment and positioning of the endoscope to the inner surface of the GI tract, i.e. to the stomach wall.

Preferably the locating pad forms the end of the bottom housing part and has an aperture present therein for the needle; and/or wherein the locating pad comprises means for attaching the end of the bottom housing part to a surface, optionally wherein the locating pad comprises at least one of a friction enhancing coating and a plurality of fibers.

By providing further means on the locating pad, this can cause the endoscope to adhere better to the surface to which it is attached, fibres or coating are beneficial kinds of ways of enhancing the attachment properties of the locating pad.

It is preferred if the magnetically actuated capsule endoscope further comprises at least one camera or camera system, wherein a first camera or camera system is provided at an end of the top housing part and is configured to record images and/or videos of an environment of the magnetically actuated capsule endoscope. In this way diagnostic images and videos of the GI tract can be obtained.

Advantageously the magnetically actuated capsule endoscope further comprises a second camera or camera system that is arranged in the region of the needle and is configured to record images and/or videos from a region around a tip of the needle. Providing a second camera that records images and videos from the region at which a biopsy is intended to be taken from, means that the action of taking the biopsy can be monitored as can the orientation of the needle, if it is noticed that the orientation of the needle is not correct or ideal via the second camera, then the endoscope can be realigned.

Preferably the magnetically actuated capsule endoscope further comprises a tether, with the tether being configured to effect a removal of the magnetically actuated capsule endoscope through the application of a force, optionally wherein the tether is configured as a conduit for electric cables configured to transmit and/or receive signals and/or energy to components, such as at least one camera or camera system, of the magnetically actuated capsule endoscope.

The tether can be a simple string by means of which the endoscope can be retrieved, e.g. via the oesophagus, by simply pulling at the tether. In other embodiments the tether can be a conduit or duct used to connect the cameras to the outside and thereby provide energy and be able to obtain the images from the cameras. It is also conceivable that the tether houses cables for further sensors that could also be provided. In this connection it should be noted that the density of tumors can differ from that of organs present in the human body, so that a sensor capable of measuring signals indicating a density could be included.

It is preferred if each of the plurality of deformable members is configured to contract on the application of a pre-defined externally applied magnetic field, preferably wherein the externally applied magnetic field induces a contraction force of greater than 0.4 N, especially of greater than 0.45 N at the plurality of deformable members start to collapse deploying the needle outside of the body.

The endoscope is moved through a workspace due to the permanent magnet present in the body thereof. To this end the external magnetic field applied to move and orientate the permanent magnet is selected in the range of 5 mT to 9 mT. In order to cause the deformable members to contract, a magnetic gradient of selected in the range of 0.5 T/m to 2 T/m is applied while a magnetic field of selected in the range of 30 mT to 70 mT is applied for orientation stabilization.

By tuning the deformable members to contract at significantly greater magnetic field strengths, an accidental release of the tip of the needle can be avoided.

Preferably each of the plurality of deformable members has an inherent restoring force of at least 0.45 N and is configured to adopt a fully expanded state when an external magnetic field is applied that induces a contraction force of less than 0.45 N at each of the plurality of deformable members. By providing the deformable members with an inherent restoring force means that if weak magnetic field gradient is applied, then the tip of the needle should be covered by the deformable members and hence the deformable members have an inherent safety function.

Advantageously the magnetically actuated capsule endoscope is configured to be moved in an externally applied magnetic field and wherein the plurality of deformable members are configured to contract on the application of a pre-defined magnetic field spatial gradient, with the magnetic field (spatial) gradient utilized for the deformation of the plurality of deformable members being larger than the magnetic field gradient used to move the magnetically actuated capsule endoscope.

It is preferred if the magnetically actuated capsule endoscope is encapsulated by a shell, with the shell being configured to dissolve in a liquid environment after a pre-defined time, with the pre-defined time preferably being selected in the range of 10 s to 10 min.

The shell is a protective layer that avoids the endoscope and in particular the tip of the needle from coming into contact with e.g. the oesophagus, as the endoscope is inserted into the human or animal body.

Preferably, in the completely uncovered state, i.e. in the fully contracted state of the deformable members, the needle tip projects by at least 5 mm, preferably 8 to 12 mm from the end of the bottom housing part.

The stomach wall has a typical thickness of 5 mm and in order to biopsy tumors lying beneath the stomach wall the needle has to penetrate not only the stomach wall, but be able to enter the tumor.

A magnetic field generating and sensing apparatus for a magnetically actuated endoscope comprising at least one permanent magnet incorporated in a magnetically actuated capsule endoscope as discussed in the foregoing, not being part of the invention, is also disclosed, the magnetic field generating and sensing apparatus comprising:

a plurality of electromagnets configured and arranged in one plane to generate a time varying magnetic force and torque on the at least one permanent magnet of the magnetically actuated endoscope, in order to effect a movement of the magnetically actuated endoscope in a workspace;
a magnetic sensor array arranged in a further plane and disposed at an opposite side of said workspace to said plurality of electromagnets;
wherein the plurality of electromagnets are configured to produce a time-varying non-uniform magnetic field;
wherein the magnetic sensor array is configured to track a position and an orientation of the magnetically actuated endoscope in the workspace; and wherein the magnetic field generating and sensing apparatus further comprises control means configured to adapt the time-varying non-uniform magnetic field in accordance with the tracked position and orientation of the magnetically actuated endoscope in order to vary at least one of the position and the orientation of the magnetically actuated endoscope.

A computer controlled electromagnetic system and magnetic sensor array were employed to control the external magnetic field precisely. Rolling locomotion and biopsy were demonstrated in vitro on a plastic stomach model with a swine adipose tissue model. After the demonstration, it was confirmed that a biopsy sample was successfully captured. Precise control techniques, the registration and mapping of the stomach, and in vivo demonstration of such a capsule robot with a biopsy tool are areas of system development remaining.

It is advantageous if the magnetic field generating and sensing apparatus further comprises a plurality of magnetic sensors that are arranged at different locations in a 2D plane. Such sensors are generally able to produce a 3D vector field map in 2D of the movement of the endoscope capsule within a target region by sensing the changes in the magnetic field.

Preferably the magnetic field generating and sensing apparatus further comprises a display apparatus and at least one input device, wherein the display apparatus illustrates images recorded by at least one camera of the magnetically actuated endoscope and/or signals output by the magnetic sensor array; and wherein a user can input commands via the at least one input device in order to actuate the control means to direct the magnetically actuated endoscope to a desired position at a desired orientation of the magnetically actuated endoscope in dependence on the illustrated images recorded by the at least one camera and/or by the magnetic sensor array. In this way a user can direct the endoscope to a target site, such as a tumor, on the basis of captured images and/or videos in an efficient way.

It is preferred if the display apparatus illustrates parameters detected by means of the plurality of magnetic sensors, i.e. a 3D vector field map in 2D of magnetic parameters.

The present teaching can also be applied to a method, not part of the invention, of actuating a magnetically actuated capsule endoscope, preferably as discussed in the foregoing, the endoscope comprising:

a body having a top housing part and a bottom housing part;
a permanent magnet arranged within the top housing part;
a plurality of deformable members extending from the top housing part to an end of the bottom housing part; and
a needle projecting from the top housing part and surrounded by the plurality of deformable members, wherein the plurality of deformable members are configured to repeatedly expand and contract to respectively cover and uncover a tip of said needle, the method comprising the steps of:

applying a magnetic field in order to move the magnetically actuated capsule endoscope through a workspace, positioning the magnetically actuated capsule endoscope at a desired location within the workspace; and changing the magnetic field to have a different field strength and/or a different magnetic field gradient in order to cause the needle tip to be repeatedly projected beyond the end of the bottom housing part.

The advantages described in the foregoing in connection with the endoscope in accordance with the invention likewise hold true for the above disclosed method.

Preferably the magnetic field applied in order to move the magnetically actuated capsule endoscope through the space has a magnetic field strength selected in the range of 5 mT to 9 mT. Advantageously, on positioning the magnetically actuated capsule endoscope at a desired location, a magnetic field having a desired strength selected in the range of 30 mT to 70 mT and a desired orientation is selected, with the desired orientation being selected in accordance with a desired direction of orientation of the needle at the desired location. Preferably the magnetic field applied to effect a deformation of the plurality of deformable members in order to cause the needle to be repeatedly moved beyond the end of the bottom housing part has a magnetic field strength selected in the range of 30 mT to 70 mT and a magnetic field inducing a force applied at the deformable members selected in the range of 0.5 to 0.8 N. These design parameters have found to yield particularly good biopsy results.

The invention will be described in detail in the following on the basis of embodiments and with reference to the Figures. These show:

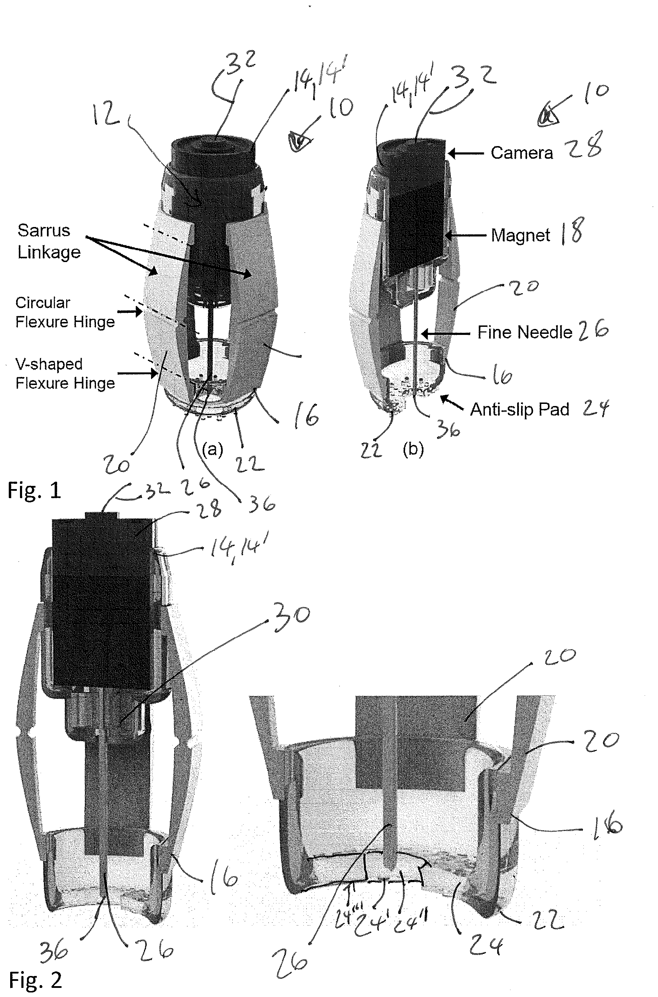

Fig. 1.  Fig. 1a exterior of a magnetically actuated capsule endoscope (B-MASCE), with the Sarrus linkage constraining the axial collapsing motion of the capsule, Fig. 1b sectional view of B-MASCE, the magnetic force induces the axial movement of the fine-needle along the Sarrus linkage, the anti-slip pad gives additional friction on the surface of the stomach;

Fig. 2  further sectional views of a magnetically actuated capsule endoscope (B-MASCE);

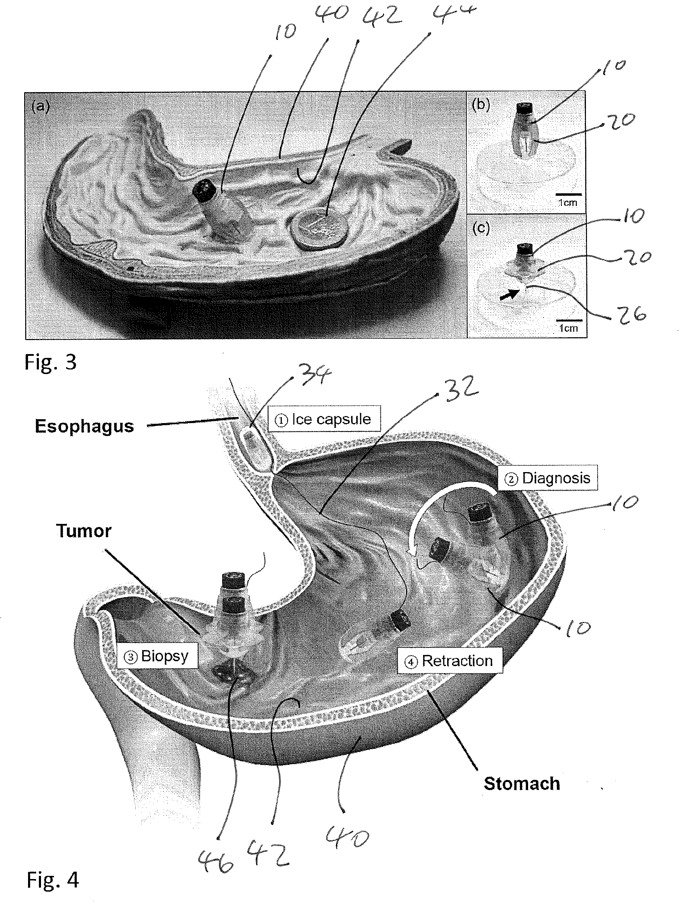

Fig. 3.  a magnetically actuated capsule endoscope (B-MASCE), with Fig. 3a illustrating B-MASCE with an anatomical stomach model and a one Eu-ro coin for size comparison and Fig. 3b showing B-MASCE facing ceiling by aligning it to an external magnetic field, Fig. 3c B-MASCE collapses with a strong gradient of the external magnetic field. The arrow indicates the exposed fine needle;

Fig. 4.  application scenario of a thin-tethered B-MASCE, with Fig. 4(1) B-MASCE is encapsulated by ice, and swallowed, Fig. 4(2) B-MASCE after the ice melts, B-MASCE can move inside the stomach and diagnose; Fig. 4(3) if a lesion is found, B-MASCE can move to the site of the lesion by rolling locomotion, and perform fine-needle aspiration biopsy, Fig. 4(4) after completing all the tasks, B-MASCE is retracted by a thin tether(Stomach illustration in background: "Gastric Ulcer" by BruceBlaus / CC BY);

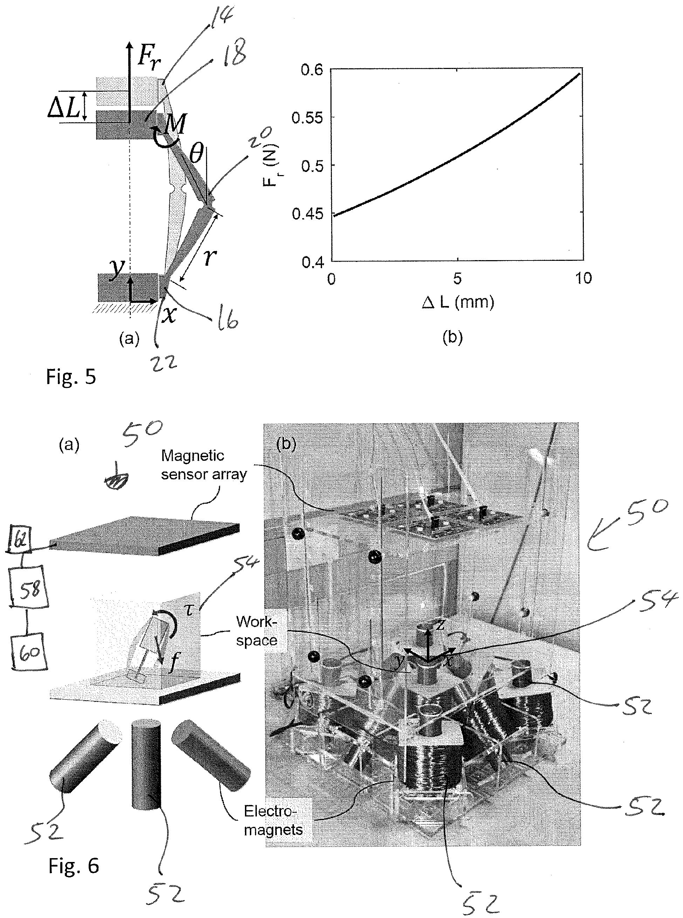

Fig. 5.  Fig. 5a diagram for the estimation of restoration force of the capsule by the penetration depth, counter forces and counter moments are omitted for reasons of visibility, Fig. 5b restoration force along the penetration depth, with the legs being designed to overcome the tissue resistance force while the needle is being retracted;

Fig. 6.  Fig. 6a a schematic diagram of the actuation system, the magnetic sensor array is placed on the top and the electromagnets are placed below, with magnetic fields from the electromagnets generating the magnetic force and torque on the magnet inside B-MASCE to move the robot inside the workspace; Fig. 6b manufactured actuation setup, sixty-four three-axis magnetic sensors are placed on the top, and nine electromagnets are placed in the bottom according to the schematic illustration, with the coordinate system showing the origin and orientation of the workspace;

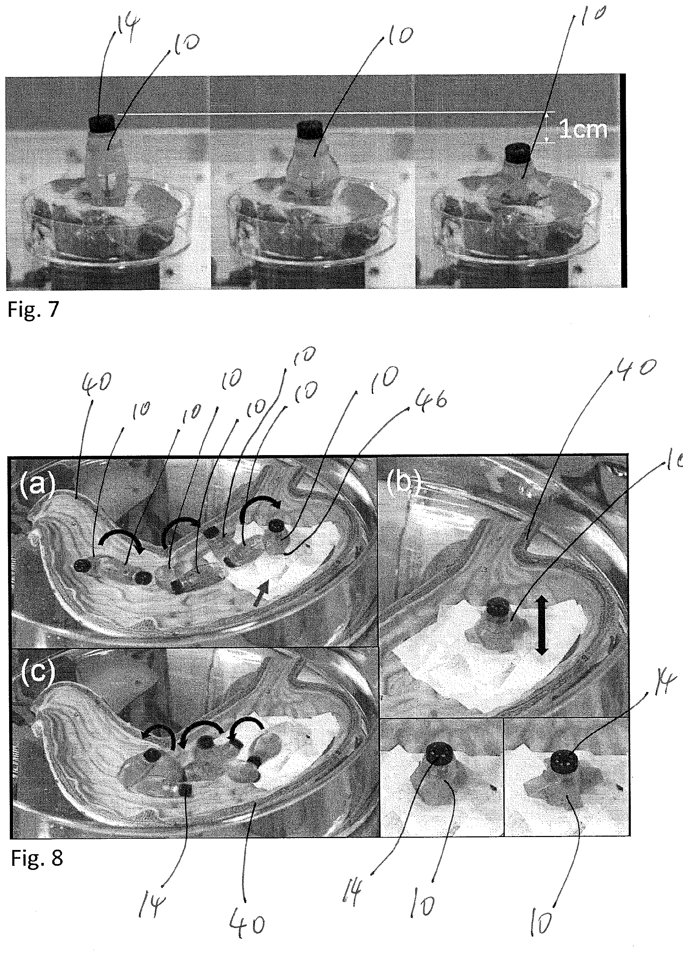

Fig. 7. collapsing motion of B-MASCE, the magnetic force causes the capsule to collapse, allowing the fine-needle to penetrate inside the tissue;

Fig. 8. demonstration of B-MASCE's rolling locomotion and the biopsy functionality in water (multiple images are overlaid), Fig. 8a B-MASCE approaches the site of a fake tumor (gray arrow) by rolling locomotion (black arrow). The pork fat is fixed with tapes due to the buoyancy, Fig. 8b BMASCE performs fine-needle biopsy with the repetitive collapsing motion (nine times), Fig. 8c B-MASCE moves to the other place waiting for the next procedure; and

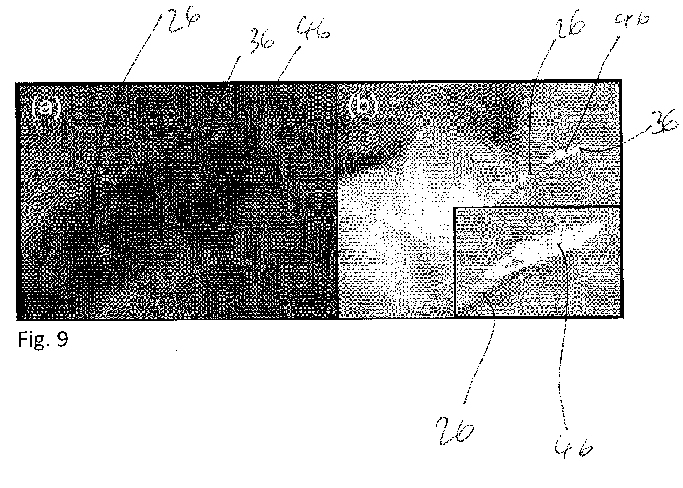

Fig. 9. biopsy sample inside the fine needle, Fig. 9a optical microscope image before discharging the tissue, the pork fat is seen inside the needle, Fig. 9b obtained sample of the pork fat, the extracted biopsy sample is observed clearly.

There are two types of fine-needle aspiration biopsy (FNAB). During the so-called suction FNAB, the fine needle is passed into the tissue and a negative pressure is applied by means of a syringe attached to the needle. The second method is called fine-needle capillary (FNC) technique, in which the needle is passed into the tissue, and five to ten successive fast jabbing movements in and out of the tissue are performed.

During FNC, the capillary force in the needle retains the detached cells inside the lumen. Clinical research has shown that there is no difference in terms of the diagnostic accuracy between the two techniques and it is recommended to use the FNC technique due to the technical ease. FNAB has better diagnostic accuracy over forceps biopsy and brush cytology in GI tract applications. Clinical research has shown that FNAB outperforms the other two techniques in upper GI application (overall diagnostic accuracy: FNAB=94%, Brush=85%, Forceps=88%). Especially in case of submucosal tumors, infiltrative malignancies, and ulceronecrotic malignancies, FNAB is significantly better as the needle can penetrate deeply into the tumor mass while the other methods only capture superficial tissues of the stomach (e.g. diagnostic accuracy for SMT: FNAB=93%, Brush=7%, Forceps=14% ).

In the following a magnetically actuated capsule endoscope (B-MASCE) will be described that is operated using the FNC technique. In this connection Fig. 1 shows the prototype of B-MASCE (a magnetically actuated capsule endoscope 10). The endoscope 10 comprises a body 12 having a top housing part 14 and a bottom housing part 16; a permanent magnet 18 arranged within the top housing part 14; a plurality of deformable members 20 extending from the top housing part 14 to an end 22 of the bottom housing part 16; and a needle 26 projecting from the top housing part 14 towards the bottom housing part 16 and surrounded by the plurality of deformable members 20. The plurality of deformable members 20 are configured to repeatedly expand and contract to respectively cover and uncover a tip 36 of said needle 26 (see also Fig. 4 in this connection). The needle 26 is accommodated in a needle hub attached to the permanent magnet 18.

The permanent magnet 18 shown in Fig. 1 is cylindrical in shape with a diameter of 8mm and a height of 8 mm. The permanent magnet in the example of Fig. 1 is made out of neodymium-iron-boron(NdFeB) 42 grade.

Generally speaking the size and strength of the permanent magnet 18 varies in dependence on the size of the capsule endoscope 10. By way of example, the diameter of the permanent magnet 18 can be selected in the range of 0.5 to 10 mm. The height of the permanent magnet 18 can be selected in the range of 1 to 20 mm. The material of the magnet can be selected such that it comprises NdFeB or similar materials.

The end 22 of the bottom housing part 16 further comprises a locating pad 24 also referred to as a friction pad 24. In Figs. 1a and b the locating pad 24 comprises a flat surface having fibers 24' present thereon. Whereas in the embodiment shown in Fig. 2 the locating pad 24 has a concave shape at the end 22 of the bottom housing part 16.

As is evident from the right hand part sectional view of Fig. 2 the locating pad 24 forms the end 22 of the bottom housing part 16 and has an aperture 24" present therein for the needle 26, i.e. on repeated contraction and expansion the top 36 of the needle 26 is moved in and out of the bottom housing part 16 via the aperture 24".

Moreover, the locating pad 24 comprises means for attaching the end 22 of the bottom housing part 16 to a surface. In the present instance the means are the plurality of fibers 24' and a friction enhancing coating 24'''.

The friction enhancing coating 24'''' could have materials as muco-adhesive, which is designed to enhance friction between the pad and mucosal surface of the gastro-intestinal tract

In the embodiment of Fig. 1 a first camera or camera system 28 is provided at the top housing part 14. The first camera or camera system 28 is configured to record images and videos of an environment of the magnetically actuated capsule endoscope 10.

The embodiment shown in Fig. 2 has a second camera or camera system 30 that is arranged in the region of the needle 26 and is configured to record images and videos from a region around the tip 36 of the needle 26. The second camera 30 is arranged at the needle hub configured to receive the needle 26. A viewing field of the second camera 30 is directed along the needle towards the tip 36 of the needle 26, i.e. along a longitudinal direction of the endoscope 10.

For the first camera or camera system 28, CMOS based high quality cameras can be used. For the second camera or camera system 30, CMOS based cameras with small footprint can be used. The second camera system might compromise the resolution or quality due to the limited space.

A tether 32 is also shown in the embodiments of Fig. 1 and 2. The tether 32 is configured to effect a removal of the magnetically actuated capsule endoscope 10 after the operations through the esophagus.

Preferably the tether 32 is further configured as a conduit for electric cables configured to transmit and/or receive signals and/or energy to components, such as the at least one camera or camera system 28, 30, of the magnetically actuated capsule endoscope 10.

The endoscope 10 typically has a length selected in the range of 20 to 32 mm, and a diameter selected in the range of 8 to 12 mm.

Preferably the material of the deformable members is selected from the group of members consisting of biocompatible elastic soft materials such as polyurethane or polydimethylsiloxane and combinations of the biocompatible elastic soft silicones.

As will be discussed in more detail in the following each of the plurality of deformable members 20 is configured to contract on the application of a pre-defined externally applied magnetic field. The externally applied magnetic field is selected such that it induces a contraction force of greater than 0.4 N, especially of greater than 0.45 N at the plurality of deformable members 20.

It should be noted in this connection that each of the plurality of deformable members 20 has an inherent restoring force of at most 0.45 N and is configured to adopt a fully expanded state when an external magnetic field is applied that induces a contraction force of less than 0.45 N at each of the plurality of deformable members 20.

It should further be noted that the magnetically actuated capsule endoscope 10 is configured to be moved in an externally applied magnetic field. The plurality of deformable members 20 are configured to contract on the application of a pre-defined magnetic field gradient, with the magnetic field gradient utilized for the deformation of the plurality of deformable members 20 being larger than the magnetic field gradient used to move the magnetically actuated capsule endoscope 10.

As is shown in Fig. 4 the magnetically actuated capsule endoscope 10 is encapsulated by a shell 34. The shell 34 is configured to dissolve in a liquid environment, such as the fluids present within the stomach after a pre-defined time. In the embodiment of Fig. 4 the shell 34 is formed by ice, but could likewise be formed by a capsule, such as those used for a time delayed release of drugs. It should be noted in this connection that the pre-defined time is selected such that one can ensure that the magnetically actuated capsule endoscope 10 remains encapsulated until it reaches the stomach 40 (see e.g. Fig. 3) of a patient. In this connection the predefined time can be being selected in the range of from 10 s to 10 min.

In the completely uncovered state, i.e. in the fully contracted state of the deformable members 20, the needle tip 36 projects by at least 5 mm, preferably at least 10 mm, from the end 22 of the bottom housing part 16.

A fine-needle 26 is attached to the top part 14 of the endoscope 10, and the capsule collapses along its longest dimension in order to deliver the needle 26 out of the bottom part 16 of the capsule.

More details of the design will be discussed in the following using the language of the inventors.

Fig. 1 shows the design features of B-MASCE 10, which is composed of a fine-needle 26, a permanent magnet 18, a four-legged Sarrus linkage 20, an upper housing 14, and a lower housing 16 with an anti-slip pattern 26. The magnet 18 exerts magnetic force and torque to the robot in response to a controlled external magnetic field. The magnetic torque is used to orient the capsule and the magnetic force is used to collapse the Sarrus linkage 20 and deliver the needle through the hole in the bottom of the capsule.

The restoration force from the Sarrus linkage 20 restores the shape of the capsule when the magnetic force is removed. Sinusoidal variation of the axial magnetic force causes repeated smooth motion of the needle 26 in and out of the tissue, which is the required motion for the FNC technique. The Sarrus linkage 20 also restricts the collapsing motion to guide the needle 26 along the axial direction of the capsule 10, which makes the motion insensitive to slight variations in the applied magnetic fields. The anti-slip pattern 24 on the bottom 16 enhances the friction during the collapsing movement to prevent unintended capsule motion prior to the needle penetration.

Table I shows the design parameters of B-MASCE 10. In this prototype, the design parameters have been selected to meet the clinical requirements and to prove initial feasibility. Clinical research has shown that 19 gauge (G) to 25 G are recommended to penetrate into tissues and get biopsy samples, and there is no performance difference in the range. For the capsule a 24 G hypodermic needle was chosen that has an outer diameter of 0.5652 mm and an inner diameter of 0.311 mm.

The average stomach wall 42 (see e.g. Fig. 3) thickness is 5.107 mm, the average upper GI tumor size is 58 mm (diameter), and the upper GI tumors are visually identifiable at sizes greater than 20 mm diameter. In the case of SMT, the needle penetration depth should be longer than the thickness of the wall, but not so long that the full thickness is pierced. The penetration depth is chosen as 10 mm to meet both criteria.

Table 1. Design parameters of the endoscope 10.

| Overall dimension | Diameter 12 mm $\times$ length 30 mm |
|---|---|
| Fine-needle | 24 G, 15 mm (length) |
| Penetration depth | 10 mm |
| Magnet NdFeB | 42 G, diameter 8 $\times$ 8 mm3 |
| Sarrus linkage: | |
| Young's modulus | 2.07 MPa |
| Leg dimension | 14 mm (H) $\times$ 6 mm (W) $\times$ 1.5 mm (D) |
| Flexure hinges | Circular: 0.275 mm (D), 0.4 mm (radius) |
| dimension | V-Shaped: 0.275 mm (D), 135° groove |

The magnet 18 must be large enough to generate sufficient magnetic force and torque. As the size of the capsule 10 is limited, the size of the magnet 18 is chosen to be the maximum size possible inside the capsule's upper body. The magnetic force and torque are controlled by the external magnetic field, which will be discussed in the following. The magnetic force should be larger than the sum of the restoration force of the Sarrus linkage 20 and the penetration resistance of the targeted tissue. The penetration resistance is different for every tissue and there is no clinical data on stomach tumor tissue. However, as the magnetic force can be adjusted easily by the external magnetic field, this is a matter of tuning for each tissue. In the current state of the research, the penetration resistance of a needle was used with a liver tissue. This is almost 0.05 N at 10 mm penetration with a 18 G (Ø1.27 mm) needle.

The Sarrus linkage leg 20 design is adopted in order to carry out the biopsy functionality, the linkage must be tuned to ensure that the combined magnetic and linkage forces provide sufficient net force for inserting and retracting the needle 26. For the design of the linkage 20, the retraction force is more critical than insertion force, because the insertion force can be safely increased by generating stronger external magnetic field gradients. However, the capsule 10 is designed to retract the needle 26 using only the restoring force of the compliant legs 20. This is done for safety; if the field gradient were used to retract the needle 26, it could cause the capsule 10 to suddenly leave the stomach wall 42, resulting in a momentary loss of control. Thus, the legs 20 were designed to be stiff enough to restore the capsule shape against the tissue's retraction resistance, which is 0.4 N at 10 mm penetration.

Fig. 3a shows the magnetically actuated capsule endoscope 10 in an anatomical stomach model 40 having a stomach wall 42. A one Euro coin 44 is included for size comparison. Fig. 3b shows B-MASCE 10 facing ceiling by aligning it to an external magnetic field. Fig. 3c shows the B-MASCE 10 in a collapsed, contracted, state collapses with a strong gradient of the external magnetic field being applied. The arrow indicates the exposed fine needle 26.

In a method of actuating the magnetically actuated capsule endoscope 10, a magnetic field is applied in order to move the magnetically actuated capsule endoscope through a workspace 54 (see Fig. 6), such as the stomach 40. The magnetically actuated capsule endoscope 10 is positioned at a desired location within the workspace 54 as shown e.g. in Fig. 3b. Once the magnetically actuated capsule endoscope 10 has been positioned at the desired location the externally applied magnetic field is changed to have a different field strength and/or a different magnetic field gradient applied at the magnetically actuated capsule endoscope 10 in order to cause the needle tip 26 to be repeatedly projected beyond the end 22 of the bottom housing part 16, i.e. to cause the needle 26 to stab the underlying tissue so that a biopsy sample 46 (see Fig. 9) can be collected in the tip 36 of the needle 26.

In this connection the magnetic field applied in order to move the magnetically actuated capsule endoscope through the space has a magnetic field strength selected in the range of 5 mT to 9 mT. The preferred motion to locate the magnetically actuated capsule endoscope through the space is a rolling locomotion.

It should further be noted that, on positioning the magnetically actuated capsule endoscope 10 at the desired location, a magnetic field having a desired strength selected in the range of 30 mT to 70 mT and a desired orientation is selected and applied. The desired orientation of the magnetically actuated capsule endoscope 10 is selected in accordance with a desired direction of orientation of the needle 26 at the desired location. This means that if e.g. a lesion or a tumor is detected by one of the cameras 28, 30, the magnetically actuated capsule endoscope 10 can be directed to the site of the lesion or tumor. In order to prepare a biopsy of the lesion or tumor the external magnetic field is then varied and applied to effect a deformation of the plurality of deformable members 20 in order to cause the needle 26 to repeatedly penetrate the tumor or lesion and in this way cause the deformable members 20 to contract and expand, so that the needle tip 36 is perceived as moving beyond the end 22 of the bottom housing part 16 and vice versa. The external magnetic field that is applied to cause the contraction has a magnetic field strength selected in the range of 30 mT to 70 mT and a magnetic field inducing a force applied at the deformable members 20 selected in the range of 0.5 to 0.8 N.

Fig. 4 shows the overall application scenario. First, BMASCE 10 is swallowed by a patient. For the protection of the esophagus, the robot 10 is capsuled by ice 34. After the ice has melted, the capsule can move around inside the stomach to visually inspect the stomach wall with the on-board camera 28, 30. The stomach 40 is insufflated by carbonated water to ensure free movement of the capsule and a clear view of the surface of the stomach 40. If a suspicious lesion is found, then B-MASCE 10 is positioned on the lesion's surface, and performs FNC. If multiple lesions are found, each can be biopsied in turn. After the operator has completed the procedure, the capsule 10 is retracted by a thin tether 32 attached to its top body 14. After the retraction, the needle 26 is taken out and attached to a syringe to remove the tissue 46 inside the needle 26. The removed tissue 46 is smeared, stained, and fixed on a microscope slide, and sent to the cytology center in a hospital.

As illustrated in Figs. 1 and 2 there are in total twelve hinges in four Sarrus linkage legs 20. The rotational stiffness of each hinge can be calculated. The upper and lower hinges are regarded as V-shaped flexure hinges and the middle one as a circular hinge. Using the values in Table I, the dimensionless rotational stiffness is expressed as

$$K = \frac{1}{Ebt^2}\left(\frac{M}{\theta}\right); K_V = 0.3513; K_C = 0.0643, \qquad (1)$$

where $K_V$ and $K_C$ are the dimensionless rotation stiffness values using the empirical fitting data from for V-shaped flexure hinges and circular hinges, respectively, E is the Young's modulus of the material, b is the depth (z-direction in Fig. 5a of the hinges), t is the minimum thickness of the hinges, $M$ is the moment on the hinges, and $\theta$ is the rotation angle. All the units are in SI unit and rad.

The compression force induces torques on the hinges, which results in angle changes in the hinges and a vertical translation of the upper body 14. Based on the kinematics of the linkages, the relationship between the penetration depth and restoration force of the capsule is expressed as

$$F_r = Ebt^2\left(8K_V\frac{\theta}{r\sin\theta} + 4K_C\frac{2\theta}{r\sin\theta}\right);$$
$$\Delta L = 2r(1 - \cos\theta) \qquad (2)$$

where $F_r$ is the restoration force. Using (2), the restoration force is plotted with the penetration depth in Fig. 5b. The restoration force is always larger than the maximum needle retraction resistive force (0.4 N, 10 mm penetration), the needle 26 will be retracted when the external magnetic force is removed. Although the current design is enough to fulfill the requirements for FNAB, it might be useful to apply magnetic shape programming techniques to the legs 20 for additional functionality.

B-MASCE 10 is fabricated with 3D-printing and molding technique. The top and bottom housings 14, 16 are 3D-printed directly (Objet260 Connex3, Stratasys co.). The Sarrus linkage legs are 3D-printed first, and used as a master part for molding. In this connection a soft polyurethane elastomer (ST-1060, BJB Enterprise Inc., Young's modulus: 2.07 MPa) was used as the final material for the legs in the prototype.

A material of the capsule endoscope 10 can for example include any soft type of elastomer that has a Young's modulus in the range of 0.5 to 5 MPa. Moreover, the capsule endoscope 10 can be coated with a variety of biocompatible sealants, for example with Polydimethylsiloxane (PDMS), Ecoflex™, UV-curable gels (Dow Corning), or similar materials.

The top housing part 14 is for example made 3D printing material, Veroclear-RGD810), and thereby forms a receptacle for the first camera 28 and for the permanent magnet 18 and possibly also for the needle hub. The locating pad 24 is e.g. made 3D printing material, Veroclear-RGD810).

A method of fabricating such legs 20 is e.g. described in S. Yim and M. Sitti, "Design and rolling locomotion of a magnetically actuated soft capsule endoscope", IEEE Transaction on Robotics, vol. 28, no. 1, pp 183-194, 2012 and in US 2013/0303847 A1, both incorporated hereby by reference having regard to the method of manufacture of the legs.

The fabricated housings 14, 16 and legs 20 are combined using cyanoacrylate glue (LOCTITE 406, Henkel Co.). The hypodermic needle 26 (24 G × 1", B. Braun Co.) is cut to 15 mm length by a nipper, and then inserted into the upper housing 14. The cut part of the needle 26 remains open. After the needle 26 is inserted, the permanent magnet 18 and a dummy camera 28 are inserted into the top housing part 14.

B-MASCE 10 is manipulated by the external magnetic field, so an accurate means of controlling the B-field and the gradient of the B-field is essential. An electromagnetic system 50 (see Fig. 6) was designed to control the magnetic field precisely and enhance the safety of the magnetic manipulation.

Fig. 6 shows a magnetic field generating and sensing apparatus 50 for a magnetically actuated endoscope 10 comprising at least one permanent magnet 18 such as the magnetically actuated capsule endoscope 10 discussed in the foregoing. The magnetic field generating and sensing apparatus 50 comprises: a plurality of electromagnets 52 configured and arranged in one plane to generate a time varying magnetic force and torque on the at least one permanent magnet 18 of the magnetically actuated endoscope 10, in order to effect a movement of the magnetically actuated endoscope 10 in a workspace 54; a magnetic sensor array 56 arranged in a further plane and disposed at an opposite side of said workspace 54 to said plurality of electromagnets 52. The plurality of electromagnets 52 are configured to produce a time-varying non-uniform magnetic field. The magnetic sensor array 56 is configured to track a position and an orientation of the magnetically actuated endoscope 10 in the workspace 54. The magnetic field generating and sensing apparatus 50 further comprises control means 62 configured to adapt the time-varying non-uniform magnetic field in accordance with the tracked position and orientation of the magnetically actuated endoscope 10 in order to vary at least one of the position and the orientation of the magnetically actuated endoscope.

The magnetic field generating and sensing apparatus 50 further comprises a display apparatus 58 and at least one input device 60, wherein the display apparatus 58 illustrates images recorded by the at least one camera 28, 30 of the magnetically actuated endoscope 10 and/or signals output by the magnetic sensor array 56.

A user can input commands via the at least one input device 60 in order to actuate the control means 62 to direct the magnetically actuated endoscope to a desired position at a desired orientation of the magnetically actuated endoscope 10 in dependence on the illustrated images recorded by the at least one camera and/or by the magnetic sensor array 56.

Thus, the schematic diagram of Fig. 6 shows the electromagnetic system and a photograph of the assembled setup. The system 50 has nine electromagnets 52 and magnetic sensor arrays 56. The electromagnets 52 are positioned at the bottom and the sensor arrays 56 are positioned at the top. They are separated by enough distance to prevent saturation of the magnetic sensors 56. The configuration of the electromagnets is optimized to generate a strong z-directional magnetic gradients, and the coils are located in a plane to keep them far enough away from the sensor system.

A design framework from OctoMag system is used for the optimization of the magnetic actuation system. However, an additional coil was added to generate a stronger B-field and field gradient, and also to allow the use of redundancy for minimization of the wasted power. In the application of the design framework, the magnetic mapping was changed into non-uniform magnetic fields and its gradient mapping, as the system uses non-uniformity of B-field. The sensor system is employed to track the position and orientation of B-MASCE. As the actuation is based on non-uniform magnetic fields, the position and orientation information of the capsule are critical to find a proper actuation mapping from the electric current to the magnetic force and torque. The sensor array 56 reads coupled magnetic fields from both B-MASCE 10 and the actuation system. Using an algorithm, the modeled magnetic field from the actuators are removed from the measured magnetic fields, and 5-D position and orientation information of the capsule 10 is calculated using a non-linear optimization algorithm in real-time.

The superposition of the magnetic field due to each coil determines the net resultant force and torque on B-MASCE's 10 permanent magnet 18. The multiple ferrous cores that are close to each other generate coupled magnetic fields. This is because a B-field from one electromagnet becomes additional H-field in the other cores, besides the original H-field from its own coil. These coupling effects should be considered in the presented setup. A multi-core system's magnetic field and force map from is used here. Using vector and matrix representations, the B-field and force are expressed as

$$\mathbf{B}_i = \frac{\mu_0}{4\pi\|\mathbf{p}_i\|^3}(3\hat{\mathbf{p}}_i\hat{\mathbf{p}}_i^\top - \mathbb{I})\mathbf{m}_i = \frac{\mu_0}{4\pi}\mathbb{P}_i\mathbf{m}_i$$

$$\mathbf{F}_i = \frac{3\mu_0}{4\pi\|\mathbf{p}_i\|^4}(\mathbf{m}\hat{\mathbf{p}}_i^\top + \hat{\mathbf{p}}_i\mathbf{m}^\top +$$

$$(\hat{\mathbf{p}}_i \cdot \mathbf{m})(\mathbb{I} - 5\hat{\mathbf{p}}_i\hat{\mathbf{p}}_i^\top))\mathbf{m}_i = \frac{3\mu_0}{4\pi}\mathbb{F}_i\mathbf{m}_i \quad (3)$$

where $\mathbf{B_i}$ and $\mathbf{F_i}$ are the B-field and force contribution from the *i-th* electromagnet, $\mu_0$ is the magnetic permeability of free space, $\mathbf{p}_i$ is the displacement vector from the *i-th* electromagnet to the center of the B-MASCE's magnet, ^ is the normalization operator to a unit vector, $\mathbb{I}$ is the 3 x 3 identity matrix, $\mathbf{m}$ is the magnetic moment of the magnet of B-MASCE, $\mathbf{m}_i$ is the magnetic moment of the *i-th* electromagnet; $\mathbb{P}_i$ and $\mathbb{F}_i$ are $3 \times 3$ matrices mapping from the magnetic moment of the *i-th* electromagnet to the B-field and force on B-MASCE's 10 magnet 18, respectively (without permeability and $\pi$ term).

The superposition of multiple electromagnets' 52 B-fields and forces are expressed in a matrix form as

$$\left[\mathbf{B}^{\top}, \mathbf{F}^{\top}\right]^{\top} = \mathbb{A}\mathbb{D}^{-1}\mathbb{M}\mathbf{I} \qquad (4)$$

where, B is the desired B-field on B-MASCE 10, F is the desired magnetic force, and I is the vector of applied currents in each coil.

$$\mathbb{A} = \frac{\mu_0}{4\pi} \begin{bmatrix} \mathbb{I} & \mathbb{O} \\ \mathbb{O} & 3\mathbb{I} \end{bmatrix} \begin{bmatrix} \mathbb{P}_1 & \cdots & \mathbb{P}_9 \\ \mathbb{F}_1 & \cdots & \mathbb{F}_9 \end{bmatrix}$$

is the actuation map which maps from the resultant magnetic moments of the electromagnets 52 to the B-field and magnetic force.

$$\mathbb{D} = \begin{bmatrix} \mathbb{I} & -\frac{V_1}{4\pi}\mathrm{N}_1\mathbb{P}_{12} & -\frac{V_1}{4\pi}\mathrm{N}_1\mathbb{P}_{13} & \cdots \\ -\frac{V_2}{4\pi}\mathrm{N}_2\mathbb{P}_{21} & \mathbb{I} & -\frac{V_2}{4\pi}\mathrm{N}_2\mathbb{P}_{23} & \\ -\frac{V_3}{4\pi}\mathrm{N}_3\mathbb{P}_{31} & -\frac{V_3}{4\pi}\mathrm{N}_3\mathbb{P}_{32} & \mathbb{I} & \\ \vdots & & & \ddots \end{bmatrix}$$

is the magnetic coupling map which encodes the coupling effects among the cores, $V_i$ is the volume of the *i-th* core, $\mathrm{N}_i$ is the *i-th* core's 3 x 3 external susceptibility matrix (in case of spherical cores: a uniform scale matrix, in the other cases: tensor rotation with directional external susceptibility values in each principal axis), and $\mathbb{P}_{ij}$ is the B-field mapping in (3) by replacing $\boldsymbol{p}_i$ to a displacement vector from *j-th* core to *i-th* core.

$$\mathbb{M} = \begin{bmatrix} \mathbf{m}_1 & \mathbf{0} & \cdots \\ \mathbf{0} & \mathbf{m}_2 & \\ \vdots & & \ddots \end{bmatrix}$$

is the packing of the magnetic moments of each electromagnets. The required electric current in each coil is achieved using the generalized (Moore-Penrose) pseudo-inverse by minimizing two-norm of the current vector as

$$\mathbf{I} = \left(\mathbb{A}\mathbb{D}^{-1}\mathbb{M}\right)^{\dagger} \left[\mathbf{B}^{\top}, \mathbf{F}^{\top}\right]^{\top}. \qquad (5)$$

Although the position and orientation information is achieved from the tracking algorithm, the direct usage of the estimated values can cause control instability due to the relatively large orientation error. The orientation of the capsule 10 can change quickly, by pivoting on the substrate, and insufficient bandwidth or accuracy in the estimation of the orientation leads to an incorrect computation since the magnetic moment of the capsule is not known accurately. In such a case, it is simpler to stabilize the system by partitioning the control; the capsule orientation is controlled by assuming that the permanent magnet 18 always aligns with the external field, and the force is controlled independently under the assumption that the orientation tracks perfectly. $\|B\| = 7\,\mathrm{mT}$ was applied with the same direction as the desired orientation of the capsule, and F = [0; 0; -0.015] N to give enough traction between the robot 10 and the substrate for rolling locomotion.

For the biopsy function, the desired B-field and force are changed due to the required strong force to collapse B-MASCE 10. A magnetic force ranging from 0.5 N to 0.8 N was applied to give enough collapsing motion over the restoration force (see Fig. 5b). For the jabbing movement using the needle 26, the applied magnetic force was sinusoidally varied within the above range of 0.5 N to 0.8 N.

Those values are determined during the first experiment to tune the fabrication errors (e.g. discrepancy of Young's modulus of legs 20, thickness of the hinges, uncertain tissue resistance, etc.). Because the very strong magnetic force is exerted on the robot 10, the desired B-field is also increased to stabilize the robot's orientation effectively. During the experiment, it was found that $\|B\| = 40$ mT is sufficient to stabilize the robot 10 while FNC is being performed.

In vitro experiments were conducted on a fresh pork fat on a plastic anatomical human stomach model (K16, 3B Scientific co.). The pork fat is chosen as a model for a GI tumor as it has a structure closer to that of a tumor than to muscle tissues. The pork fat is cut into two 25 mm diameter slices, and placed onto a Petri dish (first experiment) and into an anatomical human stomach model (demonstration see Fig. 4).

The first experiment was conducted to verify the ability of the capsule 10 to perform fine-needle aspiration biopsy. BMASCE 10 was placed on the tissue sample in the presence of a z-directional B-field, so that it could stand vertically. The gradient of the B-field was slowly increased until a full collapse was observed. The capsule 10 was placed closely to the electromagnet 52 as it requires a strong magnetic force to collapse. Although it is close to the electromagnet 52, the dipole approximation is still valid with an approximate error of 1% according to (normalized distance: 10 cm / 8 cm = 1.25, axial cylinder magnet case).

Fig. 7 shows one of the experiments on the fat tissue. It was observed that when the magnetic force is higher than the restoration force of the Sarrus linkage legs 20 (Fig. 5b), B-MASCE 10 starts to collapse. As the penetration resistance is significantly smaller than the restoration force, the penetration movement is almost the same as collapsing movement of the capsule 10 without the needle 26. It is found that 0.8N is enough to collapse B-MASCE 10 fully in the current design on the pork fat so that the needle 26 can get deep inside the tissue.

The second experiment was designed to demonstrate the concept of the application scenario. After the capsule 10 is manually placed inside of an anatomical stomach phantom, the demonstration consists of three steps: 1) move to the site of the tumor by rolling locomotion, 2) perform fine needle capillary biopsy and 3) move back to an area near the starting position. A human operator moved B-MASCE 10 by changing its orientation to induce rolling and performed biopsy changing the force along the axis of the needle. The operator observed the robot's movement and gave commands using a keyboard and a mouse on a visual interface (Lab-VIEW, National Instruments Co.). The demonstration was done under water to simulate the environment inside the water-insufflated stomach.

Fig. 8 displays the results of the demonstration. During the demonstration, the robot 10 moved relatively slowly (4 seconds for 180° turn) for stable rolling motions. Once the robot 10 had reached the tissue phantom 40 and been vertically oriented, it performed the biopsy by collapsing and recovering its shape and thereby moving the needle 26 into and out of the tissue sample.

A total of nine jabbing movements were done in 10 seconds and afterwards the capsule 10 was successfully relocated to an area near the starting position. After the demonstration, the robot 10 was retracted by hand using the tether 32 and the biopsy sample was examined by a microscope and a camera with a macro lens.

Fig. 9 shows the retracted tissue 46 from the pork fat inside the hollow needle 26. The tissue 46 was retained inside the needle 26 until the examination as a result of the capillary force. After the retraction of the capsule 10, a syringe was attached to the fine needle 26, and the sample 46 was discharged carefully by applying a positive pressure inside the syringe. From the images it is clear that the fat tissue was captured inside the needle 26. The experiment was repeated ten times, and the biopsy samples were collected on all the experiments (yield rate: 100%).

In this work, the FNC functionality using a magnetically actuated soft capsule endoscope is proposed and demonstrated. This is the first trial applying the fine-needle 26 biopsy technique to robotic capsule endoscopes 10, which enables obtaining not only superficial tissues, but also deep tissues undermining the stomach wall, such as submusosal tumors. B-MASCE has the potential to improve the diagnostic accuracy of capsule biopsy procedures in the upper GI tract, and shorten the lesion identification step by performing the diagnosis and biopsy at once.

This new design is simple, but robust to withdraw stomach tissues successfully. By utilizing the single magnet inside the robot for several purposes (locomotion, biopsy, and tracking), the number of system components is minimized, making the capsule design robust and inexpensive to fabricate. By utilizing the soft-polymer Sarrus linkages, the body of the capsule is a hinge, spring element, and encapsulating structure all-in-one. These design elements make B-MASCE significantly less complex than previously proposed biopsy capsule endoscopes, yet still able to operate reliably.

On the other hand, the safety of B-MASCE 10 needs to be considered. The robot is naturally safe due to the Sarrus linkage's restoration force. The only source which can give the collapsing force to the capsule, besides the magnetic force, is the peristalsis from the esophagus and stomach. As the esophagus is narrow, it gives constant pressure to the sides of the capsule when it is being swallowed. As a usual case, the robot 10 is aligned with the esophagus' direction due to its pill-like shape. However, there could be a little chance where the capsule is rotated inside the esophagus and be collapsed by the esophagus' peristalsis. This is the reason for the ice wall 34 surrounding the capsule 10 at the first step of the scenario by locking the shape of the capsule. For the retraction of B-MASCE 10, the tether 32 attached to the capsule plays a role. As the tether 32 is attached to the top part 14 of the capsule 10, the pulling force aligns the capsule 10 to the direction of the esophagus, which prevents B-MASCE 10 from collapsing during the retraction.

As for the stomach, the environment is different than the esophagus because the stomach is a large cavity and B-MASCE 10 is not compressed by the stomach wall. In clinical applications of capsule endoscopes 10 in the upper GI, it is recommended to drink water with air producing powder to expand the stomach as much as possible. This is for the diagnostic accuracy to see all the lesions, which can be hidden by the wrinkles of the stomach wall. In such a case, the water and the gas inside the stomach prevents the stomach from collapsing and it is safe to use B-MASCE 10.

However, there are some challenges that remain for future work: control and manipulation issues in the real environment, and registration of the stomach for presentation of the procedure status to the operator. Although the biopsy functionality was demonstrated, the test environment was significantly different than the real one. The real stomach exhibits peristalsis, which disturbs the orientation and position of the robot. In that case, a controller 62 which can robustly reject those disturbances needs to be designed and implemented. Additionally, the stomach wall is extremely slippery due to the mucosa layer. This can cause another control problem, as the pivot point of the rolling locomotion and the supporting point for the bottom pad 24 could move.

Especially if the pad 24 slips, the biopsy could be taken in an unintended location. A controller 62 which can align the robot 10 perpendicular to the surface of the stomach 40 precisely and a pad 24 which can give enough static friction need to be further developed. Additionally, the design of the actuation system is most effective when pulling the capsule's magnet towards the general direction of the iron cores. This particular arrangement is therefore not ideal for biopsy of the superior and inferior surfaces of the stomach 40. A more complex arrangement of the cores could be designed (e.g., a permanent cores case) to improve the system for this specific application.

Another necessary addition to the system is a means of registration and localization inside the stomach 40. For the demonstration, the human operator had to observe the location of the robot 10 visually, which will not be a simple task with only endoscopic video when the capsule is rolling.

Ideally, the system 50 should construct an on-line map of the stomach 40 and perform simultaneous localization and mapping to present to the operator an overview of the relative positions of the robot 10 and the stomach 40, as well as the locations of any identified lesions. The camera 28, 30 on the capsule 10 could be used to gather data for the localization and mapping procedures.

There has recently been an effort to adapt techniques for localization of mobile robots 10 to surgical scenarios inside the body, which could be incorporated into a capsule endoscopy suite for biopsy.

## Claims

1.   A magnetically actuated capsule endoscope (10), the endoscope (10) comprising:

a body (12) having a top housing part (14) and a bottom housing part (16);
a permanent magnet arranged within the top housing part (14);
a plurality of deformable members (20) extending from the top housing part (14) to an end (22) of the bottom housing part (16); **characterized in that** the endoscope further includes a needle (26) projecting from the top housing part (14) towards the bottom housing part (16) and surrounded by the plurality of deformable members (20), wherein the plurality of deformable members (20) are configured to repeatedly expand and contract to respectively cover and uncover a tip (36) of said needle (26).

2.   A magnetically actuated capsule endoscope (10) in accordance with claim 1, wherein the end (22) of the bottom housing part (16) further comprises a locating pad (24), optionally wherein the locating pad (24) has a concave shape at the end (22) of the bottom housing part (16).

3.   A magnetically actuated capsule endoscope (10) in accordance with claim 2, wherein the locating pad (24) forms the end (22) of the bottom housing part (16) and has an aperture (24") present therein for the needle (26); and/or wherein the locating pad (24) comprises means for attaching the end (22) of the bottom housing part (16) to a surface.

4.   A magnetically actuated capsule endoscope (10) in accordance with claim 3, wherein the locating pad (24) comprises at least one of a friction enhancing coating (24‴) and a plurality of fibers (24').

5.   A magnetically actuated capsule endoscope (10) in accordance with at least one of the preceding claims, further comprising at least one camera or camera system (28; 28, 30), wherein a first camera or camera system (28) is provided at an end (14') of the top housing part (14) and is configured to record images and/or videos of an environment of the magnetically actuated capsule endoscope (10).

6. A magnetically actuated capsule endoscope (10) in accordance with claim 5,
further comprising a second camera or camera system (30) that is arranged in the region of the needle (26) and is configured to record images and/or videos from a region around the tip (36) of the needle (26).

7. A magnetically actuated capsule endoscope (10) in accordance with at least one of the preceding claims,
further comprising a tether (32), with the tether (32) being configured to effect a removal of the magnetically actuated capsule endoscope (10) through the application of a force, optionally wherein the tether (32) is configured as a conduit for electric cables configured to transmit and/or receive signals and/or energy to components, such as at least one camera or camera system (28; 28, 30), of the magnetically actuated capsule endoscope (10).

8. A magnetically actuated capsule endoscope (10) in accordance with at least one of the preceding claims,
wherein each of the plurality of deformable members (20) is configured to contract on the application of a pre-defined externally applied magnetic field, optionally wherein the externally applied magnetic field induces a contraction force of greater than 0.4 N, especially of greater than 0.45 N at the plurality of deformable members (20).

9. A magnetically actuated capsule endoscope (10) in accordance with at least one of the preceding claims,
wherein each of the plurality of deformable members (20) has an inherent restoring force of at most 0.45 N and is configured to adopt a fully expanded state when no external magnetic field is applied or a partially expanded state if an external magnetic field is applied that induces a contraction force of less than 0.45 N at each of the plurality of deformable members (20).

10. A magnetically actuated capsule endoscope (10) in accordance with at least one of the preceding claims,
wherein the magnetically actuated capsule endoscope (10) is configured to be moved in an externally applied magnetic field and wherein the plurality of deformable members (20) are configured to contract on the application of a pre-defined magnetic field (spatial) gradient, with the magnetic field gradient utilized for the deformation of the plurality of deformable members (20) being larger than the magnetic field gradient used to move the magnetically actuated capsule endoscope (10).

11. A magnetically actuated capsule endoscope (10) in accordance with at least one of the preceding claims,
wherein the magnetically actuated capsule endoscope (10) is encapsulated by a shell (34), with the shell (34) being configured to dissolve in a liquid environment after a pre-defined time, optionally wherein the pre-defined time is selected in the range of 10 s to 10 min.

12. A magnetically actuated capsule endoscope (10) in accordance with at least one of the preceding claims,
wherein, in the completely uncovered state, i.e. in the fully contracted state of the deformable members (20), the tip (36) of the needle (26) projects by at least 5 mm from the end (22) of the bottom housing part (16).

**Patentansprüche**

1. Magnetisch betätigtes Kapselendoskop (10), wobei das Endoskop (10) umfasst:

    einen Körper (12) mit einem oberen Gehäuseteil (14) und einem unteren Gehäuseteil (16);
    einen Permanentmagneten, der innerhalb des oberen Gehäuseteils (14) angeordnet ist;
    eine Vielzahl von verformbaren Elementen (20), die sich von dem oberen Gehäuseteil (14) zu einem Ende (22) des unteren Gehäuseteils (16) erstrecken;
    **dadurch gekennzeichnet, dass** das Endoskop ferner umfasst:
    eine Nadel (26), die von dem oberen Gehäuseteil (14) in Richtung des unteren Gehäuseteils (16) vorsteht und von der Vielzahl von verformbaren Elementen (20) umgeben ist, wobei die Vielzahl von verformbaren Elementen (20) konfiguriert sind, um sich wiederholt auszudehnen und zusammenzuziehen, um jeweils eine Spitze (36) der Nadel (26) abzudecken und freizulegen.

2. Magnetisch betätigtes Kapselendoskop (10) nach Anspruch 1,
wobei das Ende (22) des unteren Gehäuseteils (16) ferner ein Positionierungskissen (24) umfasst, wobei das Positionierungskissen (24) optional eine konkave Form an dem Ende (22) des unteren Gehäuseteils (16) aufweist.

3. Magnetisch betätigtes Kapselendoskop (10) nach Anspruch 2, wobei das Positionierungskissen (24) das Ende (22) des unteren Gehäuseteils (16) bildet und eine darin vorhandene Öffnung (24") für die Nadel (26) aufweist; und/oder wobei das Positionierungskissen (24) ein Mittel zum Befestigen des Endes (22) des unteren Gehäuseteils (16) an einer Oberfläche umfasst.

4.  Magnetisch betätigtes Kapselendoskop (10) nach Anspruch 3, wobei das Positionierungskissen (24) mindestens eines von einer reibungsverbessernden Beschichtung (24‴) und einer Vielzahl von Fasern (24') umfasst.

5.  Magnetisch betätigtes Kapselendoskop (10) nach einem der vorhergehenden Ansprüche,
    ferner umfassend mindestens eine Kamera oder ein Kamerasystem (28; 28, 30), wobei eine erste Kamera oder ein erstes Kamerasystem (28) an einem Ende (14') des oberen Gehäuseteils (14) bereitgestellt ist und konfiguriert ist, um Bilder und/oder Videos einer Umgebung des magnetisch betätigten Kapselendoskops (10) aufzuzeichnen.

6.  Magnetisch betätigtes Kapselendoskop (10) nach Anspruch 5,
    ferner umfassend eine zweite Kamera oder ein zweites Kamerasystem (30), die/das im Bereich der Nadel (26) angeordnet ist und konfiguriert ist, um Bilder und/oder Videos von einem Bereich um die Spitze (36) der Nadel (26) aufzuzeichnen.

7.  Magnetisch betätigtes Kapselendoskop (10) nach einem der vorhergehenden Ansprüche,
    ferner umfassend ein Halteseil (32), wobei das Halteseil (32) konfiguriert ist, um eine Entfernung des magnetisch betätigten Kapselendoskops (10) durch die Anwendung einer Kraft zu bewirken, wobei optional das Halteseil (32) als eine Leitung für elektrische Kabel konfiguriert ist, die konfiguriert sind, um Signale und/oder Energie an Komponenten, wie beispielsweise mindestens eine Kamera oder ein Kamerasystem (28; 28, 30), des magnetisch betätigten Kapselendoskops (10) zu übertragen und/oder zu empfangen.

8.  Magnetisch betätigtes Kapselendoskop (10) nach einem der vorhergehenden Ansprüche,
    wobei jedes der Vielzahl von verformbaren Elementen (20) konfiguriert ist, um sich bei der Anwendung eines vordefinierten extern angelegten Magnetfelds zusammenzuziehen, wobei optional das extern angelegte Magnetfeld eine Kontraktionskraft von mehr als 0,4 N, insbesondere von mehr als 0,45 N, an der Vielzahl von verformbaren Elementen (20) induziert.

9.  Magnetisch betätigtes Kapselendoskop (10) nach einem der vorhergehenden Ansprüche,
    wobei jedes der Vielzahl von verformbaren Elementen (20) eine inhärente Rückstellkraft von höchstens 0,45 N aufweist und konfiguriert ist, um einen vollständig ausgedehnten Zustand anzunehmen, wenn kein externes Magnetfeld angelegt wird, oder einen teilweise ausgedehnten Zustand anzunehmen, wenn ein externes Magnetfeld angelegt wird, das eine Kontraktionskraft von weniger als 0,45 N an jedem der Vielzahl von verformbaren Elementen (20) induziert.

10. Magnetisch betätigtes Kapselendoskop (10) nach einem der vorhergehenden Ansprüche,
    wobei das magnetisch betätigte Kapselendoskop (10) konfiguriert ist, um in einem extern angelegten Magnetfeld bewegt zu werden, und wobei die Vielzahl von verformbaren Elementen (20) konfiguriert ist, um sich bei der Anwendung eines vordefinierten (räumlichen) Magnetfeldgradienten zusammenzuziehen, wobei der Magnetfeldgradient, der für die Verformung der Vielzahl von verformbaren Elementen (20) verwendet wird, größer ist als der Magnetfeldgradient, der verwendet wird, um das magnetisch betätigte Kapselendoskop (10) zu bewegen.

11. Magnetisch betätigtes Kapselendoskop (10) nach einem der vorhergehenden Ansprüche,
    wobei das magnetisch betätigte Kapselendoskop (10) durch eine Hülle (34) eingekapselt ist, wobei die Hülle (34) konfiguriert ist, um sich in einer flüssigen Umgebung nach einer vordefinierten Zeit aufzulösen, wobei optional die vordefinierte Zeit im Bereich von 10 s bis 10 min ausgewählt ist.

12. Magnetisch betätigtes Kapselendoskop (10) nach einem der vorhergehenden Ansprüche,
    wobei in dem vollständig freigelegten Zustand, d. h. in dem vollständig zusammengezogenen Zustand der verformbaren Elemente (20), die Spitze (36) der Nadel (26) um mindestens 5 mm von dem Ende (22) des unteren Gehäuseteils (16) vorsteht.

**Revendications**

1.  Endoscope à capsule à actionnement magnétique (10), l'endoscope (10) comprenant :

un corps (12) ayant une partie de boîtier de sommet (14) et une partie de boîtier de fond (16) ;
un aimant permanent agencé à l'intérieur de la partie de boîtier de sommet (14) ;
une pluralité d'éléments déformables (20) s'étendant depuis la partie de boîtier de sommet (14) jusqu'à une extrémité (22) de la partie de boîtier de fond (16) ;
**caractérisé en ce que**
l'endoscope inclut en outre
une aiguille (26) se projetant depuis la partie de boîtier de sommet (14) vers la partie de boîtier de fond (16) et entourée par la pluralité d'éléments déformables (20), la pluralité d'éléments déformables (20) étant configurés pour être mis, de manière répétée, en expansion et en contraction pour respectivement recouvrir et découvrir une pointe (36) de ladite aiguille (26).

2. Endoscope à capsule à actionnement magnétique (10) selon la revendication 1,
dans lequel l'extrémité (22) de la partie de boîtier de fond (16) comprend en outre un tampon de positionnement (24), en option dans lequel le tampon de positionnement (24) a une forme concave au niveau de l'extrémité (22) de la partie de boîtier de fond (16).

3. Endoscope à capsule à actionnement magnétique (10) selon la revendication 2,

dans lequel le tampon de positionnement (24) forme l'extrémité (22) de la partie de boîtier de fond (16) et a une ouverture (24") présente à l'intérieur de celui-ci pour l'aiguille (26) ;
et/ou dans lequel le tampon de positionnement (24) comprend un moyen d'attache de l'extrémité (22) de la partie de boîtier de fond (16) à une surface.

4. Endoscope à capsule à actionnement magnétique (10) selon la revendication 3,
dans lequel le tampon de positionnement (24) comprend au moins un revêtement d'amélioration de frottement (24''') et une pluralité de fibres (24').

5. Endoscope à capsule à actionnement magnétique (10) selon l'une au moins des revendications précédentes

comprenant en outre au moins une caméra ou un système de caméra (28 ; 28, 30),
dans lequel une première caméra ou un premier système de caméra (28) est prévu(e) au niveau d'une extrémité (14') de la partie de boîtier de sommet (14) et est configuré(e) pour enregistrer des images et/ou des vidéos d'un environnement de l'endoscope à capsule à actionnement magnétique (10).

6. Endoscope à capsule à actionnement magnétique (10) selon la revendication 5,
comprenant en outre une seconde caméra ou un second système de caméra (30) qui est agencé(e) dans la région de l'aiguille (26) et qui est configuré(e) pour enregistrer des images et/ou des vidéos à partir d'une région autour de la pointe (36) de l'aiguille (26).

7. Endoscope à capsule à actionnement magnétique (10) selon l'une au moins des revendications précédentes,
comprenant en outre une longe (32), la longe (32) étant configurée pour effectuer un retrait de l'endoscope à capsule à actionnement magnétique (10) via l'application d'une force, en option dans lequel la longe (32) est configurée en tant que conduit pour câbles électriques configurés pour émettre et/ou recevoir des signaux et/ou une énergie à des composants comme une caméra ou un système de caméras (28 ; 28, 30), de l'endoscope à capsule à actionnement magnétique (10).

8. Endoscope à capsule à actionnement magnétique (10) selon l'une au moins des revendications précédentes,
dans lequel chacun de la pluralité d'éléments déformables (20) est configuré pour être mis en contraction lors de l'application d'un champ magnétique prédéfini appliqué de manière externe, en option dans lequel le champ magnétique appliqué de manière externe induit une force de contraction qui est supérieure à 0,4 N, plus particulièrement qui est supérieure à 0,45 N au niveau de la pluralité d'éléments déformables (20).

9. Endoscope à capsule à actionnement magnétique (10) selon l'une au moins des revendications précédentes
dans lequel chacun de la pluralité d'éléments déformables (20) a une force de restauration inhérente d'au plus 0,45 N et est configuré pour adopter un état entièrement en expansion quand aucun champ magnétique externe n'est appliqué, ou un état partiellement en expansion si un champ magnétique externe est appliqué qui induit une force de contraction de moins de 0,45 N au niveau de chacun de la pluralité d'éléments déformables (20).

**10.** Endoscope à capsule à actionnement magnétique (10) selon l'une au moins des revendications précédentes dans lequel l'endoscope à capsule à actionnement magnétique (10) est configuré pour être déplacé dans un champ magnétique appliqué de manière externe, et dans lequel la pluralité d'éléments déformables (20) sont configurés pour être mis en contraction lors de l'application d'un gradient (spatial) de champ magnétique prédéfini, le gradient de champ magnétique utilisé pour la déformation de la pluralité d'éléments déformables (20) étant supérieur au gradient de champ magnétique utilisé pour déplacer l'endoscope à capsule à actionnement magnétique (10).

**11.** Endoscope à capsule à actionnement magnétique (10) selon l'une au moins des revendications précédentes, dans lequel l'endoscope à capsule à actionnement magnétique (10) est encapsulé par une coque (34), la coque (34) étant configurée pour se dissoudre dans un environnement liquide après une durée prédéfinie, en option dans lequel la durée prédéfinie est sélectionnée dans la plage de 10 s à 10 min.

**12.** Endoscope à capsule à actionnement magnétique (10) selon l'une au moins des revendications précédentes dans lequel, dans l'état complètement découvert, c'est-à-dire dans l'état entièrement contracté des éléments déformables (20), la pointe (36) de l'aiguille (26) se projette à raison d'au moins 5 mm depuis l'extrémité (22) de la partie de boîtier de fond (16).

Sarrus
Linkage

Circular
Flexure Hinge

V-shaped
Flexure Hinge

Camera 28

Magnet 18

Fine Needle 26

Anti-slip Pad 24

(a)    (b)

**Fig. 1**

**Fig. 2**

**Fig. 3**

**Fig. 4**

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013303847 A1
- US 2011166416 A1
- US 2011065987 A1
- JP 2008018254 A
- WO 2009060460 A2
- US 20130303847 A1

### Non-patent literature cited in the description

- **S. YIM ; M. SITTI**. Design and rolling locomotion of a magnetically actuated soft capsule endoscope. *IEEE Transaction on Robotics*, 2012, vol. 28 (1), 183-194